# DEMANDE DE BREVET EUROPEEN

(11) **EP 0 988 868 A1**
(43) Date de publication de la demande: **29.03.2000**
(21) Numéro de dépôt: 99460057.5
(22) Date de dépôt: 23.09.1999
(51) Int. Cl.: A61M 5/32, A61M 5/31

(54) **Dispositif torique d'injection sécurisé par aiguille rabattable**

(30) Priorité: 23.09.1998 FR 9812057
(71) Demandeur: Soulas, Yves, 22260 Pontrieux (FR)
(72) Inventeur: Soulas, Yves, 22260 Pontrieux (FR)

(57) **Abrégé**

L'invention concerne un dispositif permettant l'aspiration et l'injection de liquide par l'intermédiaire d'une aiguille rabattable.

Il est constitué d'un conduit torique (1) muni d'un porte-aiguille (2), dans lequel un piston (6) est mis en mouvement par un élément rotatif (5) coulissant sur les bords libres (14) du tore (1) dont il forme une partie de la paroi. L'opérateur effectue la manoeuvre en faisant tourner cet élément rotatif (5) grâce à un poussoir (7) solidaire de celui-ci.

En fin d'injection, une pièce externe (10) munie d'une zone crantée (15), coulissant autour du tore (1), vient au contact de l'élément porte-aiguille (2) par une de ses extrémités ayant forme d'entonnoir (11) et sécurise le dispositif en rabattant l'ensemble aiguille-porte-aiguille (2) qui comporte une zone de fragilité (3) à sa base, assurant ainsi son blocage.

Le dispositif selon l'invention est particulièrement indiqué dans le domaine médical afin d'éviter toute piqûre accidentelle.

## Description

La présente invention concerne un dispositif pour injecter un liquide par l'entremise d'une aiguille rabattable.

L'injection est traditionnellement effectuée au moyen d'une seringue composée d'un corps de pompe cylindrique, muni d'un embout porte-aiguille, et d'un piston.

Le dispositif selon l'invention diffère par l'instrument utilisé et le mode de fonctionnement y afférant. En effet, le principe du dispositif consiste en la poussée du liquide à injecter dans un conduit circulaire torique par l'intermédiaire d'un piston intégré à un élément central rotatif.

Le dispositif se compose de trois parties :
_ le tore, sur lequel est fixé le porte-aiguille;
_ l'élément rotatif central, comprenant le piston coulissant à l'intérieur du tore, l'ensemble constituant une paroi circulaire mobile obturant le conduit torique;
_ une pièce externe, coulissant sur l'ensemble tore-élément rotatif central, permettant de rabattre l'ensemble aiguille-porte-aiguille après l'injection, ceci étant rendu possible par la réalisation d'une zone de fragilité à la base du porte-aiguille.

Les dessins annexés illustrent l'invention :

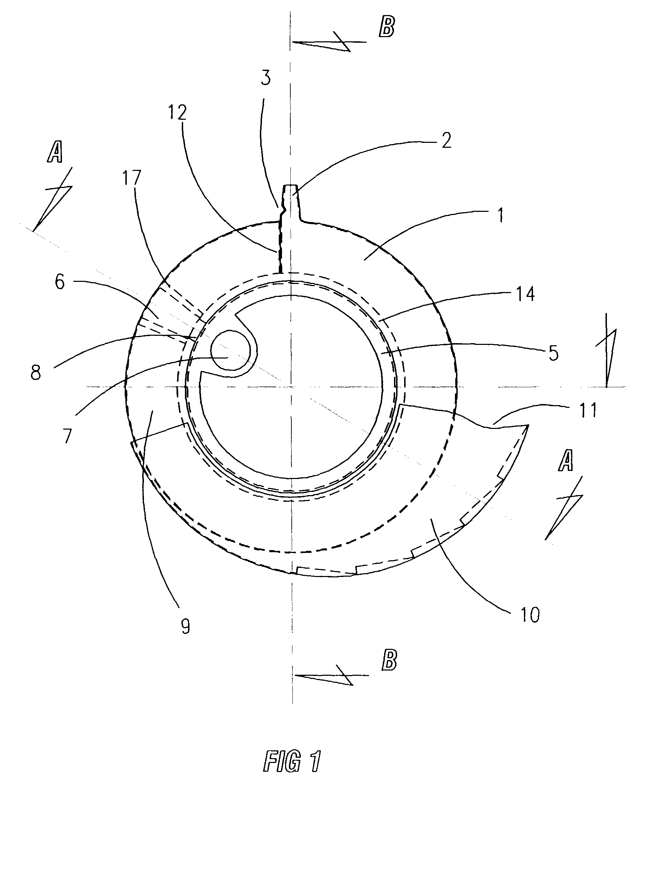

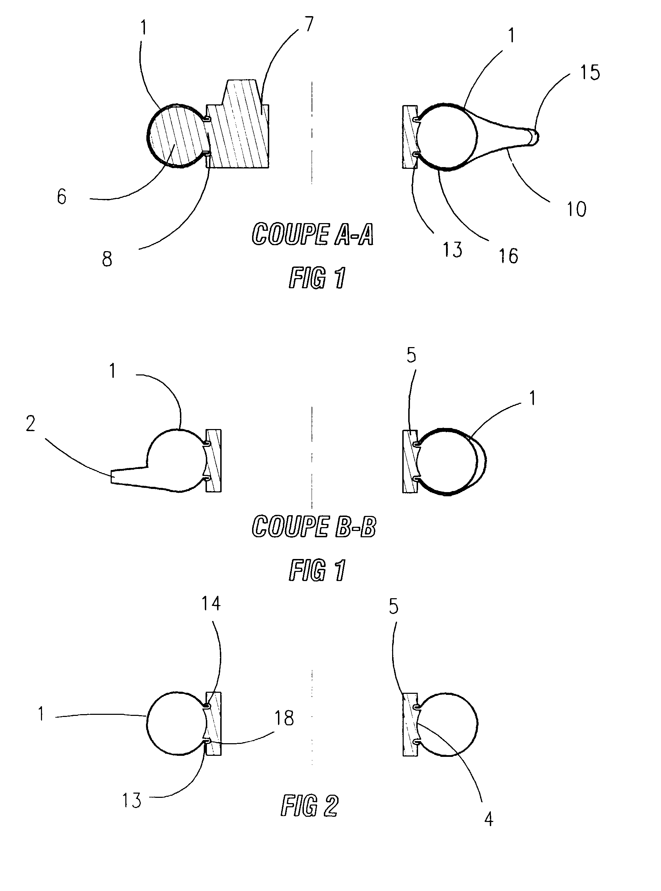

_ la figure 1 représente en coupe sagittale le dispositif selon l'invention;
_ la figure 1 selon la coupe AA représente le dispositif en coupe transversale passant par le piston (6) solidaire d'un poussoir (7) ayant dans l'exemple la forme d'un piton;
_ la figure 1 selon la coupe BB représente le dispositif, toujours en coupe transversale, muni d'un élément porte-aiguille (2) placé en position inférieure;
_ la figure 2 représente en coupe longitudinale le dispositif selon l'invention;
_ les figures 3, 4 et 5 illustrent le fonctionnement du dispositif, respectivement avant utilisation, pendant l'injection et après usage, l'ensemble des trois pièces constitutives incluant l'aiguille étant ainsi sécurisé par une impossiblité de réutilisation.
_ la figure 6 décrit le dispositif en fin d'utilisation, caractérisé en ce que la pièce externe (10) vient infléchir et rabattre l'élément porte-aiguille (2);
_ la figure 7 montre une variante du dispositif caractérisée en ce que l'élément rotatif (5) se trouve en position supérieure;
_ la figure 8 montre le poussoir (7) en position supérieure, directement au-dessus du piston (6);
_ la figure 9 décrit en coupe le détail d'un exemple d'articulation entre les éléments du dispositif coulissant entre-eux;
_ la figure 10 représente une réalisation différente du dispositif, caractérisée en ce que les bords libres (14) du tore (1) viennent recouvrir une butée (13) faisant partie de l'élément rotatif (5);
_ la figure 11 montre le dispositif en coupe transversale décrivant les différentes pièces articulées entre-elles;
_ la figure 12 représente le dispositif en coupe transversale montrant les différentes pièces articulées entre-elles, mais dans la configuration où l'élément rotatif (5) se trouve en position supérieure;
_ la figure 13 rend compte des variations possibles du positionnement de l'élément rotatif (5) vis-à-vis du tore (1);
_ la figure 14 représente la pièce externe (10) munie sur la face interne de sa courbure externe d'une zone crantée (15);
_ la figure 15 montre une variante du dispositif selon laquelle l'élément rotatif (5) s'articule tangentiellement avec le tore (1);

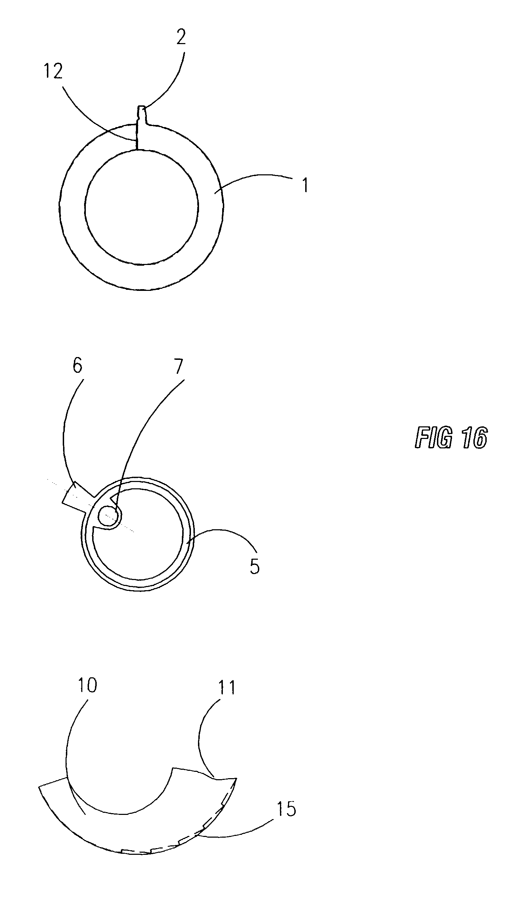

_ la figure 16 montre séparés les trois éléments du dispositif que sont :
   _ le conduit torique (1) muni de l'élément porte-aiguille (2) et d'une cloison (12),
   _ l'élément rotatif central (5) incluant d'une part le piston (6), d'autre part le poussoir (7), le tout étant moulé d'une pièce,
   _ la pièce externe (10).

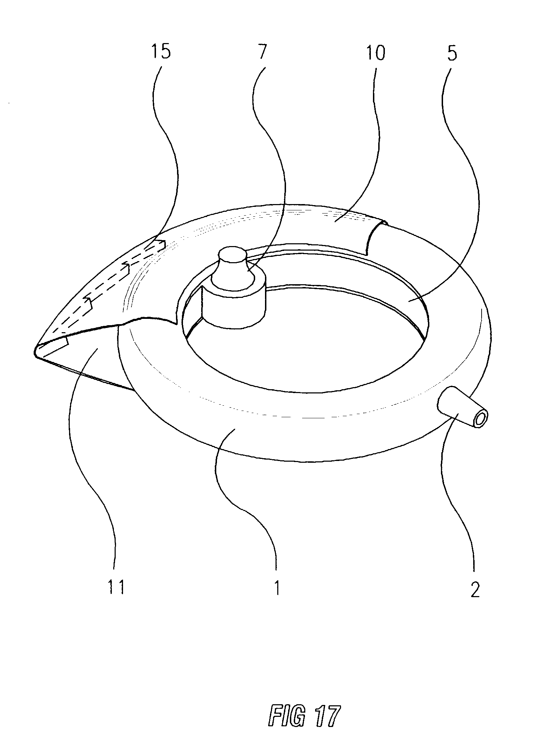

_ la figure 17 montre une représentation du dispositif dans l'espace, muni d'une pièce externe (10) comportant une zone crantée (15).

En référence à ces dessins, le dispositif comprend un conduit torique (1) sur lequel est disposé un porte-aiguille (2) muni d'une zone de fragilité à sa base (3). Ce conduit torique (1) n'est pas entièrement fermé, la partie libre (4) étant occupée par un élément rotatif central (5) venant s'y adapter, faisant office de glissière circulaire en mouvement sur les bords libres (14) du tore (1) et assurant l'étanchéité de celui-ci, selon la coupe AA de la figure 1. Une cloison (12) est disposée dans le tore (1) perpendiculairement à sa paroi, à l'aplomb du porte-aiguille (2) de telle sorte que le piston (6) vienne à son contact en expulsant le liquide (9) par l'aiguille. La cloison (12) a la forme d'un disque qui épouse la paroi interne du tore (1), à l'exception des bords libres (14) de celui-ci.

L'élément rotatif central (5) a la forme d'un anneau dont la face externe, concave vers l'extérieur de façon à compléter la section circulaire du tore (1), est parcourue sur toute sa circonférence de deux rainures formant rails (18) parallèles à son plan de rotation; dans ceux-ci viendront s'emboîter et coulisser les deux bords libres (14) du tore (1).

L'élément rotatif central (5), en un point de sa circonférence, comprend une pièce intégrant un piston (6) glissant dans le tore (1) que l'opérateur manoeuvre par l'intermédiaire d'un piton (7). Celui-ci est solidaire (8) du piston (6) qui assure la progression du liquide (9). En fin d'injection, le piston (6) vient buter sur une cloison (12), le liquide (9) étant propulsé dans l'ensemble aiguille-porte-aiguille (2).

La pièce externe (10) est constituée d'une gouttière circulaire, dont les bords libres (16) coulissent sur le tore (1); la courbure externe de cette gouttière est ainsi faite que son rayon est croissant régulièrement d'une de ses extrémités à l'autre, cette dernière formant une sorte d'entonnoir (11) qui viendra coiffer l'élément porte-aiguille (2).

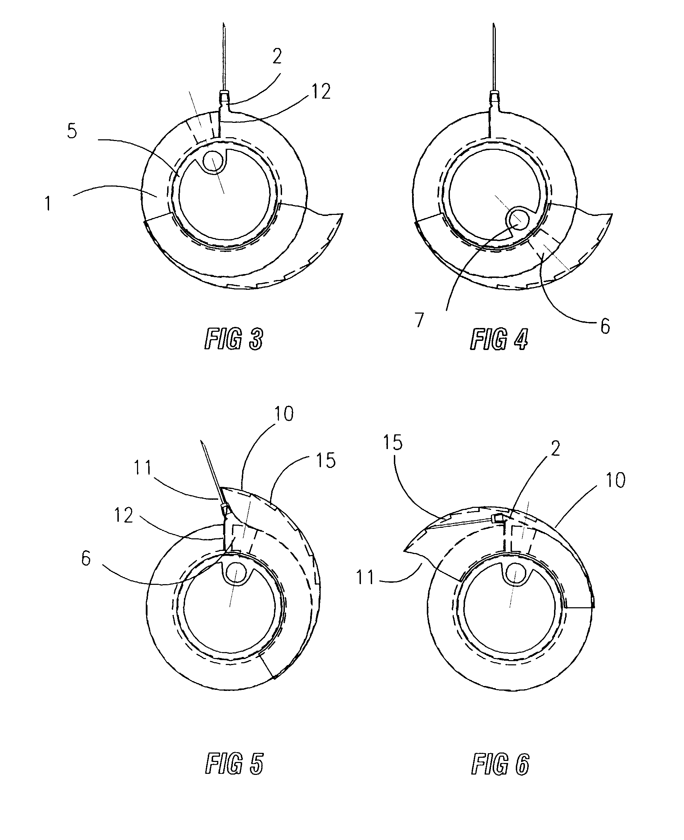

La pièce externe (10), une fois l'injection terminée, sera amenée au contact de l'ensemble aiguille-porte-aiguille (2), en coulissant sur le tore (1), selon la figure 5. La forme particulière de la pièce externe (10) permet de rabattre cet ensemble (2) dont l'inflexion est favorisée par la zone de fragilité (3). En poursuivant le déplacement de la pièce externe (10), l'opérateur provoque le recouvrement et le blocage de l'ensemble aiguille-porte-aiguille (2) dans l'espace (11) compris entre elle-même et le tore (1), l'aiguille se trouvant plaquée contre la face interne de la pièce externe (10), ainsi que le montrent les figures 5 et 6.

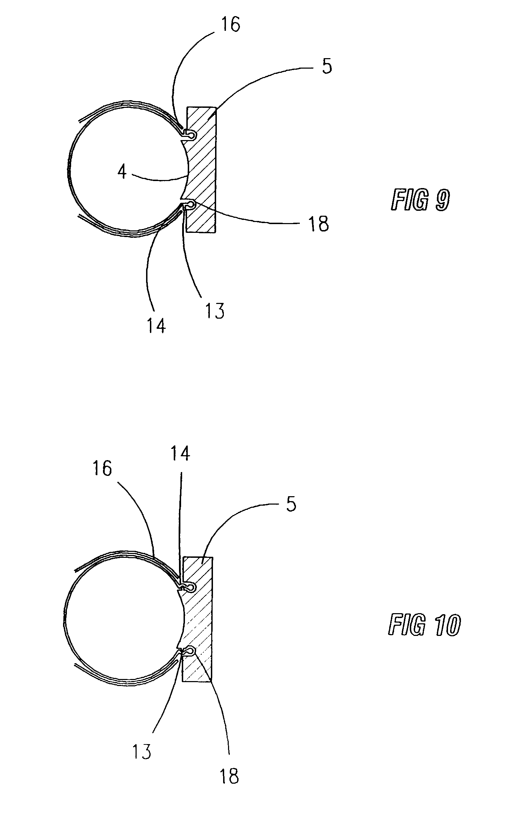

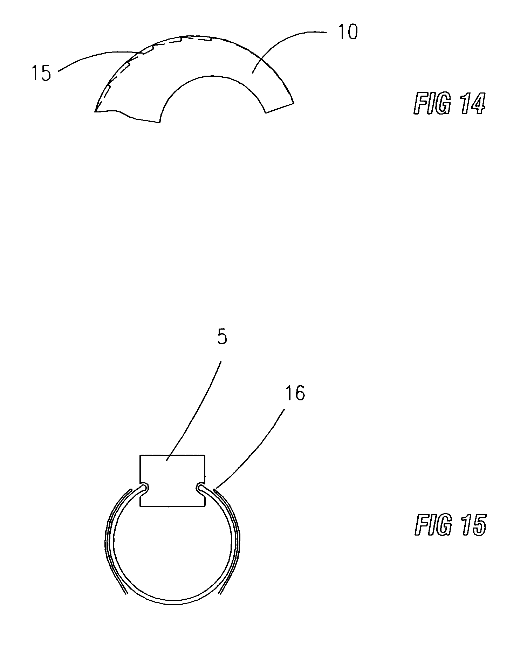

Selon des modes particuliers de réalisation :
_ le piston (6) pourra être segmenté (muni de segments (17));
_ le piston (6) pourra être muni d'un joint d'étanchéité à collerette ou segment (17) et avoir la forme d'une portion de tore;
_ le piston (6), en son extrémité, pourra avoir une forme conique;
_ la cloison (12) pourra avoir une forme conique;
_ le piton (7) pourra être amené en position supérieure, permettant de le placer à la verticale du piston (6), selon la figure 8;
_ le piton (7) pourra être réalisé par une zone creuse éventuellement rotative sur elle-même, destinée à la manipulation de l'élément rotatif central (5) par l'opérateur;
_ le piton (7) pourra avoir la forme d'un anneau.
_ l'ensemble aiguille-porte-aiguille (2) pourra être disposé à la partie inférieure du tore (1) de façon à améliorer la stabilité du dispositif lors de l'injection, selon la coupe BB de la figure 1;
_ la zone de fragilité (3) à la base de l'ensemble aiguille-porte-aiguille (2) pourra être, de façon non exclusive, composée d'une zone de moindre épaisseur;
_ la pièce externe (10) pourra au niveau de la face interne de sa courbure externe être munie d'une zone crantée (15) permettant un renforcement de cette partie de la pièce externe (10) et un blocage définitif de l'ensemble aiguille-porte-aiguille (2), selon les figures 6 et 14;
_ la pièce externe (10) pourra avoir une forme telle qu'une partie d'elle-même épousera le tore (1) tandis que la zone formant entonnoir (11) sera manipulable par l'opérateur, selon les figures 4, 5 et 6. Elle pourra disposer d'un système de blocage sur le tore (1).
_ l'articulation entre les différentes parties du dispositif pourra être définie par un système de rails (18), selon les figures 2 9 et 11; un exemple d'articulation entre les différentes parties du dispositif est ainsi détaillé suivant la figure 9 : les bords libres (14) du tore (1) viennent s'emboîter dans les rainures (18) de l'élément rotatif central (5); les bords libres (14) comprennent une partie rectiligne sur laquelle se trouve une butée en position externe (13), décrivant tout le pourtour du tore (1), réalisant en coupe un ergot; entre celui-ci et le tore (1), viennent s'insérer les bords libres (16) de la pièce externe (10).

La butée (13) pourra se trouver positionnée sur le conduit torique (1), en formant un espace faisant fonction de rails dans lesquels se déplaceront les bords libres (16) de la pièce externe (10).

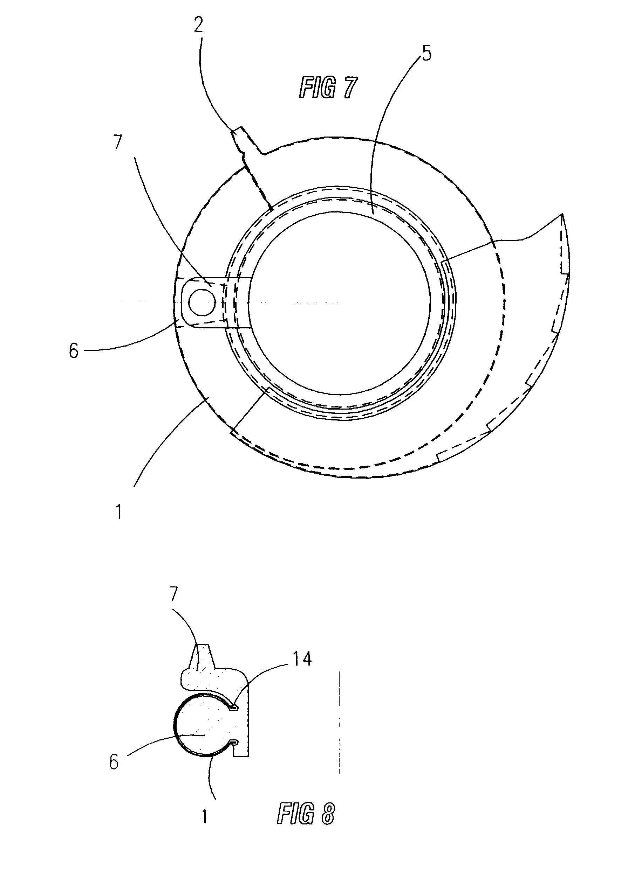

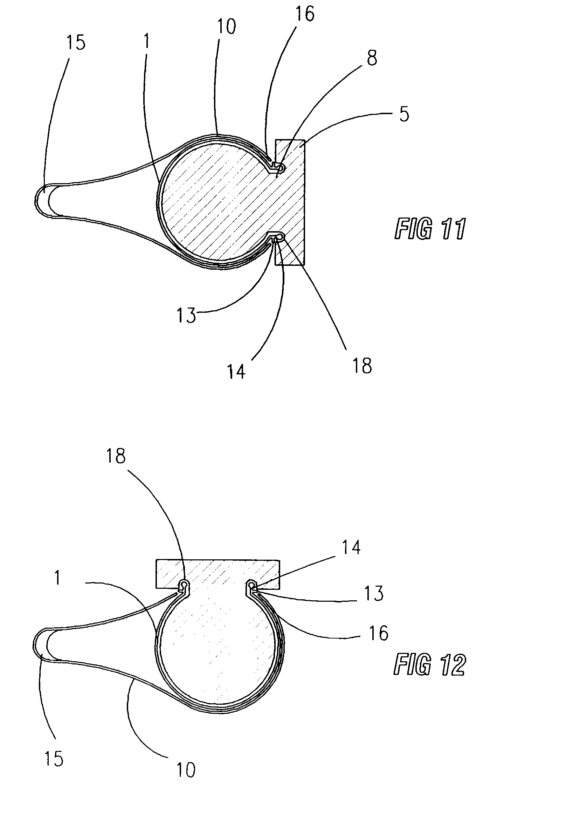

Selon une variante du dispositif, l'élément rotatif central (5) se trouve disposé à la partie supérieure du tore (1), suivant la figure 7. La pièce externe (10) est alors articulée selon le même procédé, mais dans une configuration différente, comme le met en évidence la figure 12.

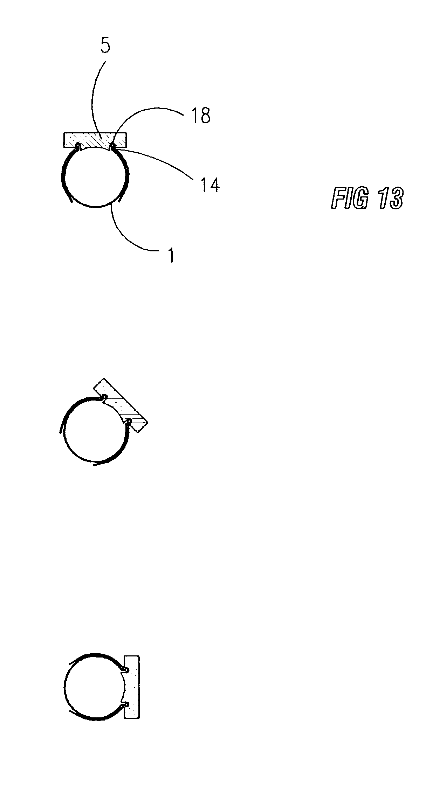

Selon une variante plus générale du dispositif, l'élément rotatif (5) pourra se positionner au niveau du cadran supéro-interne du tore (1), les deux positions extrêmes étant représentées par la configuration de cet élément rotatif (5) en position centrale et en position supérieure. La figure 13 illustre ces possibilités.

Selon une autre variante du dispositif, l'élément rotatif central (5) s'articule de manière tangentielle avec les bords libres (14) du tore (1), suivant la figure 15.

A titre d'exemple, le dispositif pourra avoir un diamètre extérieur de 6 à 8 cm, de façon à s'adapter de manière optimale à la main de l'opérateur ou de l'opératrice, le calibre du tore étant fonction de la contenance voulue et du volume de liquide à injecter, après étalonnage et graduation de ce tore à l'instar des seringues traditionnelles.

L'étanchéité de l'ensemble pourra être obtenue grâce à un compromis entre congruence et mobilité des pièces entre elles par le choix des matériaux et l'utilisation éventuelle de joints, sous la forme de film imperméable constitué de résine synthétique.

Le dispositif selon l'invention est indiqué dans le domaine médical, particulièrement dans les conditions d'utilisation requérant une impossibilité absolue de contamination par piqûre accidentelle. Sa simplicité de conception (3 pièces pouvant être réalisées en matériaux plastiques, transparents), la présence d'une pièce externe qui, en rendant le dispositif non réutilisable, conditionne son usage unique, sa possibilité d'adaptation de manière simple à un dispositif d'entraînement électrique analogue aux pousse-seringues électriques actuels, autorisent son développement industriel pour un faible coût.

## Revendications

1. Dispositif pour injecter un liquide par le biais d'une aiguille caractérisé en ce qu'il comporte un conduit torique (1) sur lequel coulisse un élément rotatif (5), pourvu d'un porte-aiguille (2) et dans lequel est mis en mouvement un piston (6).

2. Dispositif selon la revendication 1 caractérisé en ce qu'une pièce externe (10) coulisse autour du tore (1) et vient rabattre l'ensemble aiguille-porte-aiguille (2) et le bloquer.

3. Dispositif selon la revendication 1 et la revendication 2 caractérisé en ce que l'ensemble aiguille-porte-aiguille (2) est rabattable grâce à une zone de fragilité (3) à la base de celui-ci.

4. Dispositif selon la revendication 3 caractérisé en ce que la face interne de la courbure externe de la pièce externe (10) comporte une zone crantée (15) permettant de bloquer l'aiguille.

5. Dispositif selon la revendication 1 caractérisé en ce que la progression du liquide à aspirer ou à injecter s'effectue par l'entremise d'un poussoir (7) solidaire (8) du piston (6), le tout étant partie intégrante de l'élément rotatif (5).

6. Dispositif selon la revendication 1 caractérisé en ce que l'élément rotatif central (5) est parcouru de deux rails parallèles (18) dans lesquels coulissent les bords libres (14) du tore (1).

7. Dispositif selon les revendications 1, 2 et 5 caractérisé en ce que le conduit torique (1) présente une butée (13) déterminant avec celui-ci un espace formant rail, aux parties inférieure et supérieure du tore (1), dans lequel coulissent les bords libres (16) de la pièce externe (10).

8. Dispositif selon les revendications 2, 3, 4 et 7 caractérisé en ce que la pièce externe (10) est formée d'une zone interne épousant le conduit torique (1) et d'une partie externe s'évasant à une de ses extrémités, formant un espace (11) qui fait entonnoir.

9. Dispositif selon la revendication 5 caractérisé en ce que le piton (7) est rotatif sur sa base afin de faciliter la manipulation de l'élément rotatif central (5) et par conséquent du piston (6) dont il fait partie intégrante.
